# EUROPEAN PATENT SPECIFICATION

(11) **EP 4 474 816 B1**
(45) Date of publication and mention of the grant of the patent: **19.08.2026**
(21) Application number: 24180068.9
(22) Date of filing: 04.06.2024
(51) Int. Cl.: G01N 33/08

(54) **EGG ASSESSMENT DEVICE**
EIBEWERTUNGSVORRICHTUNG
DISPOSITIF D'ÉVALUATION D' UFS

(30) Priority: 08.06.2023 NL 2035040
(43) Date of publication of application: 11.12.2024
(73) Proprietor: Van de Ven Beheer B.V., 5521 DZ Eersel (NL)
(72) Inventor: VAN DE VEN, Dick Hendrikus Cornelis, 5521NB Eersel (NL); HOFLAND, Luc, 6922EN Duiven (NL)
(74) Representative: Verhees, Godefridus Josephus Maria

(56) References cited:
- NL-B1- 2 025 764

## Description

### Technical field of the invention

The invention relates to an egg assessment device comprising:
- lighting means for illuminating an egg to be assessed,
- optical recording means for recording, during illumination of a first side of the egg, an image of a second side of the egg to be assessed opposite the first side,
- assessment means for assessing the egg on the basis of the recorded image of the exposed egg,
- a light source, which illuminates a place, where an egg to be assessed should be present, from one side,
- light measuring means to measure light from the light source for detecting the presence of an egg to be assessed, and
- control means for controlling the lighting means.

Quality detection is an important aspect of the production process in the egg processing industry. It is important for both the laying sector and the hatching sector that broken eggs (for example a crack in the shell) are removed from the process before the eggs are packaged or used for hatching (reduced chance of hatching). This quality detection is partly based on visual assessment.

The assessment tools indicate if an egg has been rejected and this egg must then be removed from the process. The assessment means are based on analysis of the recorded image and may contain a self-learning algorithm in which a human inspector indicates when an assessment has been incorrect.

### Background of the invention

An egg assessment device according to the preamble of claim 1 is known from NL2025764B1. This known egg assessment device is provided with detection means for detecting whether an egg is present in an egg holder or not and, in case of presence of an egg in this egg holder, switch on the lighting means, which during assessment of this egg, illuminate this egg from below and in the absence of an egg in this egg holder does not switch on these lighting means. This known system does not work flawlessly but has a margin of error. A percentage of the approved eggs should have been rejected.

### Summary of the invention

It is an object of the present invention to provide an egg assessment device of the type described in the opening paragraph with which the quality of individual eggs can be better assessed. To this end, the egg assessment device according to the invention is characterized in that:
- the light measuring means are luminous intensity measuring means comprising a light meter, which is present on a further side opposite said side illuminated by the light source, for measuring the luminous intensity of the transmitted light passing through the egg, and
- the control means are intensity adjustment means that are configured for adjusting the intensity of the lighting means depending on the brightness of the transmitted light measured by the light meter.

In the known device according to NL2025764B1 the light measuring means are configured to only detect the presence or absence of light whereas in the device according to the invention the light measuring means are intensity measuring means configured to measure the intensity of the light transmitted through an egg. Further, in the known device according to NL2025764B1 the control means are configured to only switch the lighting means on or off whereas the in the device according to the invention the control means are intensity adjustment means configured to adjust the intensity of the lighting means.

Research has shown that the margin of error in the assessment of eggs is largely caused by the fact that the recorded image of some eggs appears to be overexposed and of others underexposed, despite the fact that the eggs are all exposed in the same way. The difference in results turned out to be due to the light transmittance of eggs. Because the light transmittance of eggs appears to vary and the eggs are illuminated from one side and an image is recorded from the opposite side, it has been found that the image of highly translucent eggs is overexposed and that the image of relatively poorly translucent eggs is underexposed. The difference in light transmittance is mainly determined by the structure of the eggshell of eggs. This effect appears to be greatest for brown eggs and hatching eggs.

By measuring the light transmittance of eggs and adjusting the lighting intensity accordingly, the eggs are prevented from being over- or under-exposed during the visual inspection, to prevent that cracks or other damage or deviations to the egg are not detected during the visual inspection. By ensuring that the lighting results in the same brightness of the image for all eggs and that the assessment tools are adjusted accordingly, the chance of errors arising during the assessment is greatly reduced, so that the assessment performance is improved.

The light intensity of the images of eggs is therefore optimized per individual egg. The result of this is that various deviations, such as hairline cracks, fractures, contamination, irregular surfaces ('mottling'), etc., are better recognized.

An embodiment of the egg assessment device according to the invention is characterized in that the egg assessment device further comprises moving means for moving the eggs from a supply position to a light transmittance measuring position where the light transmittance of the egg to be assessed is determined and from there to an assessment position where the quality of the egg is assessed on the basis of an optical assessment and then to an output position. By having the light transmittance determination and assessment take place in different places, different light sources can be used for both functions to better optimize the lighting. Furthermore, this makes it possible to simultaneously assess an egg and determine the light transmittance of a subsequent egg, so that more eggs can be assessed per unit of time.

A further embodiment of the egg assessment device according to the invention is characterized in that the moving means comprise transport means for moving the egg to be assessed along a conveyor path.

Yet another embodiment of the egg assessment device according to the invention is characterized in that the moving means further comprise rotation means for rotating the egg to be assessed so that different parts of the outside of the egg can be assessed.

The rotation means preferably comprise rollers that are moved along the conveyor track and rotate during movement so that an egg present thereon is rotated and can be assessed at various locations on the outside.

In order to be able to assess eggs simultaneously, the transport means comprise at least one further transport track, which is parallel to the said transport track and the egg assessment device comprises further lighting means, further light transmittance measuring means and further adjustment means for the lighting intensity. Furthermore, in this case the egg assessment device preferably comprises further rotation means so that different eggs can also be rotated simultaneously for assessment at different locations on the outside.

In order to reduce adverse mutual influence on measurements and assessments, the further light transmittance measuring means preferably comprise a further light source, wherein the light sources of the light transmittance measuring means and the further light transmittance measuring means alternately provide a light pulse for illumination.

In order to reduce mutual influence even further, the light sources of the light transmittance measuring means and of the further light transmittance measuring means, viewed in the direction of transport of the eggs, are preferably offset with respect to each other, so that the eggs from two adjacent paths are not simultaneously above the light sources of the light transmittance measuring means and of the further light transmittance measuring means.

Yet another embodiment of the egg assessment device according to the invention is characterized in that the egg assessment device further comprises presence detection means for detecting the presence of an egg at the location of the light source, wherein the light source only emits light in the presence of an egg. If no egg is present and the light source is active, the light emitted will adversely affect other measurements or assessments that are taking place elsewhere due to the absence of an egg. This is prevented by only activating the light source when an egg is present. When activated, the light source preferably emits a short-lived light pulse so that other measurements are affected as little as possible during illumination.

### Brief description of the drawings

The invention will be explained in more detail below on the basis of an exemplary embodiment of the egg assessment device according to the invention shown in the drawings. In the drawings:
Figure 1 is a schematic representation of the egg assessment device in side view; and
Figure 2 is a top view of the transport means and lighting means of the egg assessment device.

### Detailed description of the drawings

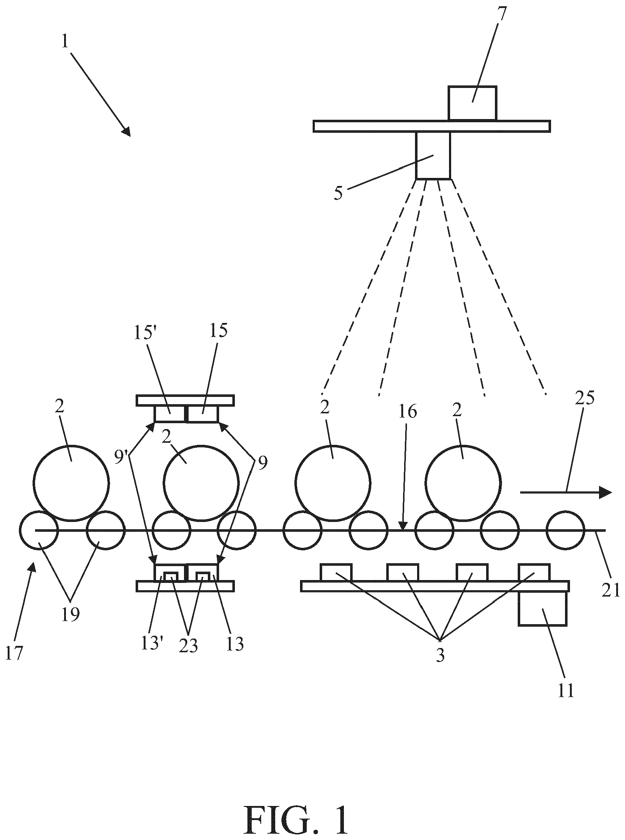

Figure 1 shows a schematic side view of an embodiment of the egg assessment device according to the invention. The egg assessment device 1 has lighting means 3 for illuminating eggs 2 to be assessed from below. These lighting means 3 are formed by LEDs. The egg assessment device 1 further has optical recording means 5 formed by a camera for recording an image of the eggs to be assessed during the illumination of the eggs 2. This camera is located at a distance above the eggs to be assessed and makes an image of all eggs present at that moment above the lighting equipment.

The image of the eggs 2 recorded by the camera is subjected to assessment means 7 formed by a computer programmed to analyze the recorded image and to recognize broken and damaged eggs from it. The rejected eggs must then be removed.

The egg assessment device 1 further has light transmittance measuring means 9 for measuring the light transmittance of the individual eggs 2 to be assessed. If an egg is more translucent, the lighting intensity of the egg must be reduced to prevent overexposure. Conversely, the lighting intensity must be increased if an egg is less translucent. The lighting means 3 are connected to adjustment means 11 which adjust the intensity of the lighting means 3 for that egg depending on the measured light transmittance.

The light transmittance measuring means 9 are formed by a light source 13 (LED) that illuminates the egg 2 to be assessed from below and a light meter 15 (LDR) that measures the light intensity of the transmitted light. This light meter 15 is located on the top side of the egg 2 opposite the light source 13.

The egg assessment device 1 further has moving means for moving the eggs 2 from a supply position (far left in figure 1) to a position where the light transmittance is determined and from there to a zone where the quality is assessed on the basis of an optical assessment and then to an output position (far right in Figure 1). During the optical assessment, each egg is assessed at four different locations, distributed over the circumference of the egg 2. To this end, the lighting means 3 are formed by four LEDs that are spaced behind each other in the direction of movement 25.

The moving means for moving the eggs are composed of transport means 16 for moving eggs 2 in a straight conveyor path 21 and of rotation means 17 for turning the eggs about the central axis. The rotation means have rollers that are displaced in the direction of transport and also rotate about their axis. The eggs lie in the space between two rollers present one behind the other and are carried and rotated by these rollers.

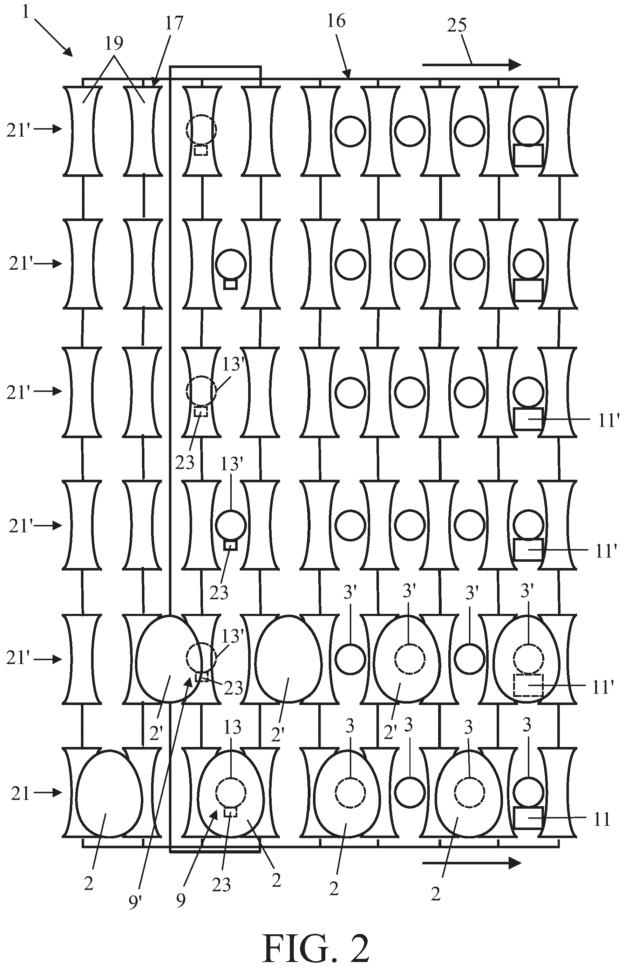

Figure 2 shows the transport means and the lighting means of the egg assessment device in top view. There are six parallel conveyor belts 21 and 21', six LEDs 13 and 13' next to each other for illuminating the eggs during the light transmittance measurement and a matrix of six by four LEDs 3 and 3' for illuminating the eggs during the visual assessment of eggs 2 and 2'. Each conveyor track 21, 21' also contains lighting means 3, 3', light transmittance measuring means 9, 9', adjustment means 11, 11' for the lighting intensity and rotation means 17, 17' for rotating the further egg 2'.

Light sources 13, 13' of the light transmittance measuring means 9, 9', which are present next to each other, are staggered relative to each other, viewed in the transport direction 25 of the eggs, and alternately emit a light pulse for the purpose of illumination. This means that the measurements are not disturbed by stray light from light sources in neighboring conveyors.

The egg assessment device 1 further has presence detection means 23 for detecting the presence of an egg at the location of the light sources 13 and 13', whereby the light sources only emit light in the presence of an egg to limit stray light as much as possible.

Although the present invention is elucidated above on the basis of the given drawings, it should be noted that this invention is not limited whatsoever to the embodiments shown in the drawings. The invention also extends to all embodiments deviating from the embodiments shown in the drawings within the scope of the invention defined by the appended claims.

## Claims

1. Egg assessment device (1) comprising:
- lighting means (3) for illuminating a first side of an egg (2) to be assessed,
- optical recording means (5) for recording, during the illumination of the egg (2), an image of a second side of the egg to be assessed opposite the first side,
- assessment means (7) for assessing the egg (2) on the basis of the recorded image of the exposed egg,
- a light source (13), which illuminates a place, where an egg to be assessed should be present, from one side,
- light measuring means (9) to measure light from the light source (13) for detecting the presence of an egg to be assessed, and
- control means (11) for controlling the lighting means (3),
**characterized in that**:
- the light measuring means (9) are luminous intensity measuring means comprising a light meter (15), which is present on a further side opposite said side illuminated by the light source (13), for measuring the luminous intensity of the transmitted light passing through the egg, and
- the control means (11) are intensity adjustment means that are configured for adjusting the intensity of the lighting means (3) depending on the luminous intensity of the transmitted light measured by the light meter (15).

2. Egg assessment device according to any one of the preceding claims, **characterized in that** the egg assessment device (1) further comprises moving means for moving the eggs (2) from a supply position to a light transmittance measuring position where the light transmittance of the egg to be assessed is determined and from there to an assessment position where the quality of the egg is assessed based on an optical assessment and then to an output position.

3. Egg assessment device according to claim 2, **characterized in that** the moving means comprise transport means (16) for moving the egg (2) to be assessed along a conveyor track (21).

4. Egg assessment device according to claim 2 or 3, **characterized in that** the moving means further comprise rotation means (17) for rotating the egg (2) to be assessed, so that different parts of the outside of the egg can be assessed.

5. Egg assessment device according to claim 4, **characterized in that** the rotation means (17) comprise rollers (19) that are moved along the conveyor track and rotate during movement so that an egg present thereon is rotated and assessed at various locations on the outside.

6. Egg assessment device according to claim 4 or 5, **characterized in that** for assessing a further egg (2') the conveying means comprise at least one further conveyor track (21'), which is parallel to said conveyor track (21) and that the egg assessment device (1) comprises further lighting means (3'), further light transmittance measuring means (9') and further adjustment means (11') for the lighting intensity.

7. Egg assessment device according to claim 6, **characterized in that** the egg assessment device (1) comprises further rotation means (17') for turning the further egg (2').

8. Egg assessment device according to claim 6 or 7, **characterized in that** the further light transmission measuring means (9') comprise a further light source (13'), wherein the light sources (13, 13') of the light transmission measuring means (9) and the further light transmission measuring means ( 9') alternately give a light pulse before illuminating.

9. Egg assessment device according to claim 8, **characterized in that** the light sources (13, 13') of the light transmission measuring means (9) and of the further light transmission measuring means (9') are offset from each other, viewed in the direction of transport (25) of the eggs.

10. Egg assessment device according to any one of the preceding claims, **characterized in that** the egg assessment device (1) further comprises presence detection means (23) for detecting the presence of an egg at the location of the light source, wherein the light source only emits light in the presence of an egg.

## Patentansprüche

1. Eierprüfgerät (1) umfassend:
- Beleuchtungseinrichtung (3) zur Beleuchtung einer ersten Seite eines zu prüfenden Eis (2),
- optische Aufzeichnungseinrichtung (5) zur Aufzeichnung eines Bildes der, der ersten Seite gegenüberliegenden, zweiten Seite des zu prüfenden Eis während der Beleuchtung des Eis (2),
- Prüfeinrichtung (7) zur Prüfung des Eis (2) anhand des aufgezeichneten Bildes des belichteten Eis,
- eine Lichtquelle (13), die einen Bereich, in dem sich ein zu prüfendes Ei befinden soll, von einer Seite beleuchtet,
- eine Lichtmesseinrichtung (9) zur Messung des Lichts der Lichtquelle (13) zum Nachweis des Vorhandenseins eines zu prüfenden Eis und
- eine Steuereinrichtung (11) zur Steuerung der Beleuchtungseinrichtung (3),
**dadurch gekennzeichnet, dass**:
- die Lichtmesseinrichtung (9) eine Lichtstärkemesseinrichtung ist, bestehend aus einem Lichtmesser (15), der sich auf der der von der Lichtquelle (13) beleuchteten Seite gegenüberliegenden Seite befindet und die Lichtstärke des durch das Ei hindurchtretenden Lichts misst, und
- die Steuereinrichtung (11) eine Intensitätsregelung ist, die die Intensität der Beleuchtungseinrichtung (3) in Abhängigkeit von der mit dem Lichtmesser (15) gemessenen Lichtstärke des durchgelassenen Lichts anpasst.

2. Eierprüfgerät nach einem der vorhergehenden Ansprüche, **dadurch gekennzeichnet, dass** das Eierprüfgerät (1) ferner eine Bewegungseinrichtung zum Transport der Eier (2) von einer Zufuhrposition zu einer Lichtdurchlässigkeitsmessposition, an der die Lichtdurchlässigkeit des zu prüfenden Eis bestimmt wird, und von dort zu einer Prüfposition, an der die Qualität des Eis anhand einer optischen Prüfung beurteilt wird, und anschließend zu einer Ausgabeposition umfasst.

3. Eierprüfgerät nach Anspruch 2, **dadurch gekennzeichnet, dass** die Bewegungseinrichtung eine Transporteinrichtung (16) zum Transport des zu prüfenden Eis (2) entlang einer Förderbahn (21) umfasst.

4. Eierprüfgerät nach Anspruch 2 oder 3, **dadurch gekennzeichnet, dass** die Bewegungseinrichtung ferner eine Dreheinrichtung (17) zum Drehen des zu prüfenden Eis (2) umfasst, sodass verschiedene Bereiche der Eieraußenseite geprüft werden können.

5. Eierprüfvorrichtung nach Anspruch 4, **dadurch gekennzeichnet, dass** die Dreheinrichtung (17) Rollen (19) umfasst, die sich entlang der Förderbahn bewegen und während der Bewegung drehen, sodass ein darauf befindliches Ei gedreht und an verschiedenen Stellen an der Außenseite geprüft wird.

6. Eierprüfvorrichtung nach Anspruch 4 oder 5, **dadurch gekennzeichnet, dass** die Fördereinrichtung zur Prüfung eines weiteren Eis (2') mindestens eine weitere Förderbahn (21') umfasst, die parallel zur Förderbahn (21) verläuft, und dass die Eierprüfvorrichtung (1) weitere Beleuchtungseinrichtungen (3'), weitere Lichtdurchlässigkeitsmessgeräte (9') und weitere Einstelleinrichtungen (11') für die Beleuchtungsintensität umfasst.

7. Eierprüfvorrichtung nach Anspruch 6, **dadurch gekennzeichnet, dass** die Eierprüfvorrichtung (1) weitere Dreheinrichtungen (17') zum Drehen des weiteren Eis (2') umfasst.

8. Eierprüfvorrichtung nach Anspruch 6 oder 7, **dadurch gekennzeichnet, dass** die zusätzliche Lichttransmissionsmesseinrichtung (9') eine zusätzliche Lichtquelle (13') umfasst, wobei die Lichtquellen (13, 13') der Lichttransmissionsmesseinrichtung (9) und der zusätzlichen Lichttransmissionsmesseinrichtung (9') abwechselnd einen Lichtimpuls abgeben, bevor sie leuchten.

9. Eierprüfvorrichtung nach Anspruch 8, **dadurch gekennzeichnet, dass** die Lichtquellen (13, 13') der Lichttransmissionsmesseinrichtung (9) und der zusätzlichen Lichttransmissionsmesseinrichtung (9') in Transportrichtung (25) der Eier zueinander versetzt sind.

10. Eierprüfvorrichtung nach einem der vorhergehenden Ansprüche, **dadurch gekennzeichnet, dass** die Eierprüfvorrichtung (1) ferner eine Anwesenheitserkennungseinrichtung (23) zur Erkennung des Vorhandenseins eines Eis am Ort der Lichtquelle umfasst, wobei die Lichtquelle nur dann Licht emittiert, wenn sich ein Ei in der Nähe befindet.

## Revendications

1. Dispositif d'évaluation d'œufs (1) comprenant :
- un moyen d'éclairage (3) pour illuminer une première face d'un œuf (2) à évaluer,
- un moyen d'enregistrement optique (5) pour enregistrer, pendant l'illumination de l'œuf (2), une image d'une seconde face de l'œuf à évaluer, opposée à la première face,
- un moyen d'évaluation (7) pour évaluer l'œuf (2) sur la base de l'image enregistrée de l'œuf exposé,
- une source lumineuse (13) qui illumine d'un côté l'emplacement où doit se trouver un œuf à évaluer,
- un moyen de mesure de la lumière (9) pour mesurer la lumière émise par la source lumineuse (13) afin de détecter la présence d'un œuf à évaluer, et
- un moyen de commande (11) pour contrôler le moyen d'éclairage (3),
**caractérisé en ce que** :
- le moyen de mesure de la lumière (9) est un moyen de mesure de l'intensité lumineuse comprenant un luxmètre (15), situé sur la face opposée à celle éclairée par la source lumineuse (13), pour mesurer l'intensité lumineuse de la lumière transmise à travers l'œuf, et
- les moyens de commande (11) sont des moyens de réglage d'intensité configurés pour ajuster l'intensité des moyens d'éclairage (3) en fonction de l'intensité lumineuse de la lumière transmise, mesurée par le luxmètre (15).

2. Dispositif d'évaluation des œufs selon l'une quelconque des revendications précédentes, **caractérisé en ce que** le dispositif d'évaluation des œufs (1) comprend en outre des moyens de déplacement pour acheminer les œufs (2) d'une position d'alimentation vers une position de mesure de la transmittance lumineuse où la transmittance lumineuse de l'œuf à évaluer est déterminée, puis vers une position d'évaluation où la qualité de l'œuf est évaluée par une analyse optique, et enfin vers une position de sortie.

3. Dispositif d'évaluation des œufs selon la revendication 2, **caractérisé en ce que** les moyens de déplacement comprennent des moyens de transport (16) pour déplacer l'œuf (2) à évaluer le long d'un convoyeur (21).

4. Dispositif d'évaluation des œufs selon la revendication 2 ou 3, **caractérisé en ce que** les moyens de déplacement comprennent en outre des moyens de rotation (17) pour faire tourner l'œuf (2) à évaluer, permettant ainsi d'évaluer différentes parties de sa surface extérieure.

5. Dispositif d'évaluation d'œufs selon la revendication 4, **caractérisé en ce que** les moyens de rotation (17) comprennent des rouleaux (19) qui se déplacent le long du convoyeur et tournent pendant leur déplacement, de sorte qu'un œuf présent sur celui-ci soit mis en rotation et évalué à différents endroits de sa surface extérieure.

6. Dispositif d'évaluation d'œufs selon la revendication 4 ou 5, **caractérisé en ce que**, pour évaluer un autre œuf (2'), les moyens de convoyage comprennent au moins un autre convoyeur (21'), parallèle audit convoyeur (21), et que le dispositif d'évaluation d'œufs (1) comprend des moyens d'éclairage supplémentaires (3'), des moyens de mesure de la transmittance lumineuse supplémentaires (9') et des moyens de réglage supplémentaires (11') de l'intensité lumineuse.

7. Dispositif d'évaluation d'œufs selon la revendication 6, **caractérisé en ce que** le dispositif d'évaluation d'œufs (1) comprend des moyens de rotation supplémentaires (17') pour faire tourner l'œuf supplémentaire (2').

8. Dispositif d'évaluation des œufs selon la revendication 6 ou 7, **caractérisé en ce que** les moyens de mesure de la transmission lumineuse supplémentaires (9') comprennent une source lumineuse supplémentaire (13'), les sources lumineuses (13, 13') des moyens de mesure de la transmission lumineuse (9) et des moyens de mesure de la transmission lumineuse supplémentaires (9') émettant alternativement une impulsion lumineuse avant de s'allumer.

9. Dispositif d'évaluation des œufs selon la revendication 8, **caractérisé en ce que** les sources lumineuses (13, 13') des moyens de mesure de la transmission lumineuse (9) et des moyens de mesure de la transmission lumineuse supplémentaires (9') sont décalées l'une par rapport à l'autre, dans le sens du transport (25) des œufs.

10. Dispositif d'évaluation des œufs selon l'une quelconque des revendications précédentes, **caractérisé en ce que** le dispositif d'évaluation des œufs (1) comprend en outre des moyens de détection de présence (23) permettant de détecter la présence d'un œuf à l'emplacement de la source lumineuse, cette dernière n'émettant de lumière qu'en présence d'un œuf.
